# EUROPÄISCHE PATENTSCHRIFT

(11) **EP 0 835 114 B1**
(45) Veröffentlichungstag und Bekanntmachung des Hinweises auf die Patenterteilung: **07.05.2003**
(21) Anmeldenummer: 96922739.6
(22) Anmeldetag: 27.06.1996
(51) Int. Cl.: A61K 31/585, A61K 31/57, A61K 31/565

(54) **PHARMAZEUTISCHES KOMBINATIONSPRÄPARAT, KIT UND METHODE ZUR HORMONALEN KONTRAZEPTION**
PHARMACEUTICAL COMBINED PREPARATION, KIT AND METHOD FOR HORMONAL CONTRACEPTION
PREPARATION PHARMACEUTIQUE COMBINEE, NECESSAIRE ET METHODE DE CONTRACEPTION ORALE

(30) Priorität: 28.06.1995 DE 19525017
(43) Veröffentlichungstag der Anmeldung: 15.04.1998
(73) Patentinhaber: SCHERING AKTIENGESELLSCHAFT, 13353 Berlin (DE)
(72) Erfinder: SCHMIDT-GOLLWITZER, Karin, D-14129 Berlin (DE); KLEMANN, Walter, D-14052 Berlin (DE)
(86) Internationale Anmeldenummer: DE9601192
(87) Internationale Veröffentlichungsnummer: WO97001342

(56) Entgegenhaltungen:
- WO-A-92/07589
- WO-A-95/07081
- DE-A- 4 344 405
- DE-A- 4 411 585
- BUNDESVERBAND D. PHARM. IND. E.V.: "Rote Liste 1995" 1995 , EDITIO CANTOR , AULENDORF/WÜRTT. XP002019696 siehe Nr.75072: " Primosiston"
- BUNDESVERBAND D. PHARM. IND. E.V.: "Rote Liste 1995" 1995 , EDITIO CANTOR , AULENDORF/WÜRTT. XP002019697 siehe Nr. 75135: "Oviol 22 / Oviol 28" siehe Nr. 75138: "Sequostat"

## Beschreibung

Die vorliegende Erfindung betrifft ein zweistufiges pharmazeutisches Kombinationspräparat zur hormonalen Kontrazeption enthaltend mindestens 30 tägliche Dosierungseinheiten, welches in seiner ersten Stufe als hormonellen Wirkstoff in Kombination ein Estrogen- und in mindestens zur Ovulationshemmung ausreichender Dosierung ein Gestagenpräparat in einstufiger Ausbildung und in der zweiten Stufe als hormonellen Wirkstoff lediglich ein Estrogenpräparat enthält, wobei die erste Stufe mindestens 25 und maximal 77 tägliche, diskret oder kontinuierlich applizierte Dosierungseinheiten und die zweite Stufe 5, 6 oder 7 tägliche, diskret oder kontinuierlich applizierte Dosierungseinheiten umfaßt und die Gesamtzahl der Tageseinheiten gleich der Gesamtzahl der Tage des gewünschten, mindestens 30 und maximal 84 Tage langen, Zyklus ist,
sowie eine entsprechende, dieses Kombinationspräparat enthaltende Packung (kontrazeptionelles Kit) und
eine kontrazeptive Methode, die sich des vorstehenden Kontrazeptionspräparates bedient.

Orale Kontrazeptiva in Form von Kombinationspräparaten sind als sogenannte Einphasenpräparate seit 1960 bekannt. Diese Präparate bestehen aus 21 wirkstoffhaltigen Dosierungseinheiten und 7 wirkstofffreien Tabletten oder Dragees. Die tägliche Dosierungseinheit ist aus einem Estrogen und Gestagen zusammengesetzt. In Einphasenpräraraten ist die täglich zu verabreichende Dosis der aktiven Stoffe in jeder Dosierungseinheit gleich hoch. Wenn die täglich zu verabreichende Dosis der aktiven Bestandteile in den einzelnen Dosierungseinheiten in einzelnen Abschnitten über den Verabreichungszyklus unterschiedlich ist, handelt es sich um sogenannte Mehrphasenpräparate. Als besonders namhafter Vertreter sei Triquilar® genannt
(DE-A 23 65 103).

Durch die Entwicklung neuer, wirksamerer Gestagene als die in den ersten oralen Kontrazeptiva enthaltenen, konnte die tägliche Gestagendosierung kontinuierlich verringert werden. Auch die tägliche Estrogendosierung konnte gesenkt werden, obwohl als Estrogen in hormonalen Kontrazeptiva nach wie vor meist Ethinylestradiol enthalten ist.
Bei der Entwicklung neuer, verbesserter oraler Kontrazeptiva standen (und stehen) folgende drei Gesichtspunkte im Vordergrund:
Es soll
(1) die kontrazeptive Sicherheit,
(2) eine gute Zykluskontrolle, d.h. geringe Inzidenz an Zwischenblutungen und
(3) ein Minimum an unerwünschten Nebenwirkungen
gewährleistet sein.
Die kontrazeptive Sicherheit wird vor allem durch die Gestagenkomponente bewirkt. Deren tägliche Dosierungsmenge enspricht jeweils mindestens der Grenzdosis, die für das betreffende Gestagen zur Ovulationshemmung als erforderlich angesehen wird. Das in Kombinationspräparaten als Estrogen meistens verwendete Ethinylestradiol soll den ovulationshemmenden Effekt des Gestagens erhöhen und vor allem die Zyklusstabilität gewährleisten. Die tägliche Dosis bei alleiniger Verabreichung des Ethinylestradiols, die für eine Hemmung der Ovulation verwendet werden muss, beträgt 100 µg.

Kombinationspräparate mit der jüngsten Generation von Gestagenen sind z.B. die Einphasenpräparate Femovan (DE-PS 2546062) oder Marvelon (DE-OS 2361120).
Als Mehrphasenpräparat, dessen Dosierungseinheiten ein Gestagen der jüngsten Generation, nämlich Gestoden, enthalten, ist beispielsweise Milvane® zu nennen (EP- 0 148 724). Bei diesen Dreiphasenpräparaten werden in der ersten Phase zumeist 4-6 Dragees verabreicht, in der jedes Dragee eine Estrogenmenge in geringer Dosis und ein Gestagen in geringer Dosis enthält. In der zweiten Phase von 4-6 Dragees enthält jede Dosierungseinheit ein Estrogen mit gleicher oder gering angehobener, maximal bis auf das 2 fache gesteigerte Dosis und ein Gestagen mit gleicher oder gering angehobener, maximal auf das 1.5 fach gesteigerte Dosis. In einer dritten Phase von 9-11 Einheiten enthält jedes Dragee ein Estrogen mit gleicher oder wieder gesenkter, maximal auf den Ausgangswert erniedrigter Dosis und ein Gestagen mit weiter angehobener, maximal auf das 3 fache des Ausgangswertes gesteigerter Dosis. Daran schliessen sich 7 pillenfreie Tage an
Neuerdings sind auch mehrphasische Kombinationspräparate vorgeschlagen worden, die eine verlängerte. d.h. bis zu 24tägige Einnahme wirkstoffhaltiger Dosierungseinheiten im 28tägigen Zyklus vorsehen können. Dabei steigt die tägliche Gestagen-Dosierungsmenge von der ersten über die zweite bis zur dritten Phase entweder an (EP-A 0 491 415) oder sie nimmt ab (EP-A 0 491 438). Zur Vervollständigung des 28 Tage langen Zyklus schließen sich im ersten Fall 4 Blindpillentage, 4 Placebos oder aber 4 ausschließlich gestagenhaltige Dosierungseinheiten oder im zweiten Fall 4 bis 7 Blindpillentage oder 4 bis 7 Placebos an.

Die Entwicklung neuer oraler Kontrazeptiva mit verringerter täglicher Hormondosis hatte zum Ziel, die in epidemologischen Studien beschriebenen Nebenwirkungen zu minimieren. Neuere epidemologische Daten weisen auf einen solchen Trend zur besseren Verträglichkeit niedriger dosierter Präparate hinsichtlich kardiovaskulärer Nebenwirkungen hin [ Thorogood M, Oral Contraceptives and Cardiovascular Disease: An Epidemiologic Overview; Pharmacoepidemiology and Drug Safety, Vol 2: 3-16 (1993); Gerstman B B, Piper J M, Tomita D K, Ferguson W J, Stadel B V, Lundin F E; Oral Contraceptive Estrogen Dose and the Risk of Deep Venous Thromboembolic Disease, Am J E, Vol. 133, No 1, 32-36 (1991); Lidegaard O, Oral contraception and rist of a cerebral thromboembolic attack: results of a case-control study; BMJ Vol 306, 956-63 (1993); Vessey M, Mant D, Smith A, Yeates D., Oral contraceptives and venous thromboembolism: findings in a large prospective study; BMJ, Vol 292, (1986); Mishell D R, Oral Contraception: Past, Present, and Future Perspectives; Int J Fertil, 36 Suppl., 7 - 18 (1991)].

Ein Zusammenhang zwischen der Höhe der täglichen Estrogendosis und der Häufigkeit kardiovaskulärer Komplikationen wird angenommen.

Das Präparat mit der zur Zeit niedrigst dosierten Estrogenmenge ist als Mercilon® im Handel und enthält 20µg Ethinylestradiol in Kombination mit 150µg Desogestrel in jeder täglichen Dosierungseinheit über 21 Tage mit einem sich anschließenden 7 tägigem pillenfreien Intervall. Die Zykluskontrolle dieses Präparates ist im Vergleich zu Präparaten mit höherer Estrogendosis erwartungsgemäß etwas schlechter. Ein weiteres klinisch bedeutsames Problem stellt die in mehreren Studien übereinstimmend gemachte Beobachtung einer geringeren ovariellen Suppression des 20µg Ethinylestradiol enthaltenden Präparates dar. Es kommt offensichtlich unter dieser sehr niedrigen Estrogendosis bei vielen Frauen zur Heranreifung von Follikeln, die mit Ultraschalluntersuchungen bzw. Hormonuntersuchungen nachgewiesen werden konnten [Lunell N O, Carlström K, Zador G, Ovulation inhibition with a combined oral contraceptive containing 20 µg ethinylestradiol and 250 µg levonorgestrel; Acta Obstet Gynecol Scand Suppl. 88: 17-21 (1979); Mall-Haefeli M, Werner-Zodrow 1, Huber P R, Klinische Erfahrungen mit Mercilon und Marvelon unter besonderer Berücksichtigung der Ovar-Funktion; Geburtsh. und Frauenheilk. 51, 35-38, Georg Thieme Verlag, Stuttgart-New York (1991); Strobel E, Behandlung mit oralen Kontrazeptiva; Fortschr. Med. 110 Jg. Nr. 20 (1992); Letter to Editor, Contraception 45: 519-521 (1992); Teichmann A T, Brill K, Can Dose Reduction of Ethinylestradiol in OCs jeopardize Ovarian Suppression and Cycle Control? Abstract Book, VIIIth World Congress on Human Reproduction, Bali, Indonesia (1993)].

Bis vor kurzem wurde eine mehrtägige Unterbrechung der Einnahme wirkstoffhaltiger Dragees für notwendig gehalten, um eine Entzugsblutung auszulösen und eine ausreichende Zykluskontrolle zu gewährleisten.

Andere Präparate wurden beschrieben, die einen estrogenen und gestagenen Wirkstoff enthalten und die im allgemeinen über 21 Tage in gleichbleibenden Mengen in jeder einzelnen Dosierungseinheit verabreicht werden, bei denen der Einnahme dieser einen estrogenen und gestagenen Wirkstoff enthaltenden Dosierungseinheiten die Einnahme ausschließlich Estrogen-haltiger Dosierungseinheiten (Ijzerman, US-A 3,502,772; Pasquale, US-A 4,921,843; Kuhl et al., EP-A 0 499 348) vorausgeht. Bei diesen Präparaten wird bei Einnahmebeginn entweder bereits am ersten Zyklustag (Kuhl) oder frühestens am zweiten Zyklustag (Pasquale) mit der Einnahme von Dosierungseinheiten begonnen, die nur einen estrogenen Wirkstoff enthalten, und zwar in einer Dosierung, die unter der ovulationshemmenden Dosis der estrogenen Komponente liegt, wodurch es, insbesondere im ersten Einnahmezyklus, zu Follikelentwicklungen kommen kann. Follikelentwicklungen werden fiir Durchbruchsovulationen verantwortlich gemacht (Chowdhury et al., "Escape" ovulation in women due to the missing of low dose combination oral contraceptive pills, Contraception, 22: 241-247, 1980; Molloy B.G. et al., "Missed pill" conception: fact or fiction? Brit.Med.J. 290, 1474-1475, 1985). Der kontrazeptive Schutz ist dadurch in Frage gestellt. Das Risiko einer Schwangerschaft ist daher insbesondere bei Einnahmefehlern unter den 20µg-Ethinylestradiol Präparaten hoch.

Die DE-OS 43 13 926 ein mindestens vierphasiges pharmazeutisches Präparat zur Kontrazeption aus einer fixen oder sequentiellen, mindestens dreiphasigen Kombination von Ethinylestradiol oder Mestranol oder einem anderen synthetischen Estrogen sowie einem Gestagen und einem mindestens in der vierten Phase des Zyklus zu applizierenden Estrogenpräparat, das Ethinylestradiol oder Mestranol und/oder ein anderes synthetisches und/oder körpereigenes Estrogen enthält. Dieses Präparat ist über den gesamten Zyklus der Frau zu verabreichen und ist auf eine 28tägige Verabreichung beschränkt.

Allen bisher am Markt befindlichen Präparaten zur hormonalen Kontrazeption ist gemein, daß deren jeweilige Verpackungseinheit auf einen 28-tägigen Einnahmezyklus ausgerichtet ist (4-Wochenrythmus). Für einen einjährigen, d.h. 12-monatigen, Verhütungszeitraum ist also die Einnahme des Inhalts von 13 Verpackungseinheiten erforderlich.

Es ist allerdings bereits seit langer Zeit bekannt, daß sich der Eintritt der Menstruation unter der Einnahme eines oralen Kontrazeptivums durch kontinuierliche tägliche Einnahme sowohl estrogen- als insbesondere gestagenhaltiger Dosierungseinheiten bis zur Beendigung der Einnahme der gestagenhaltigen Dosierungseinheiten hinausschieben läßt [Hamerlynck J. V. Th. H. et al., Contraception 35,3: 199-205 (1987); Luodon N. B., IPPF Med Bull 13,1: 2-3 (1979); Luodon N. B. et al., Brit Med J 60085: 487-490 (1977/2)]. Nach dem Absetzen der gestagenhaltigen Dosierungseinheiten tritt eine Entzugsblutung auf. Das ovulationshemmende Mittel gemäß der EP-A 0 499 348 ist zwar nicht auf einen gewünschten Einnahmezyklus von 28 Tagen beschränkt, jedoch bleibt dort die Anzahl der sowohl estrogen- als auch gestagenhaltigen Hormontageseinheiten auf eine Obergrenze von 23 Tageseinheiten beschränkt.

Die Aufgabe der vorliegenden Erfindung ist es, ein Kombinationspräparat mit möglichst niedrigem Estrogengehalt in jeder einzelnen Dosierungseinheit aber auch mit einem niedrigen Gesamthormongehalt pro Verabreichungszyklus zur Verfügung zu stellen, wobei bei hoher kontrazeptiver Sicherheit bereits auch im ersten Einnahmezyklus eine möglichst geringe Inzidenz an Follikelentwicklung, eine einwandfreie Zykluskontrolle unter zuverlässiger Vermeidung von Zwischenblutungen wie Durchbruchblutungen und "spottings" erreicht und unerwünschte Nebenwirkungen vermieden werden sollen.

Diese Aufgabe wird durch die Bereitstellung des eingangs beschriebenen zweistufigen Kombinationspräparates sowie eine entsprechende. dieses Kombinationspräparat enthaltende Packung (kontrazeptionelles Kit) und eine kontrazeptive Methode, die sich des beschriebenen Kontrazeptionspräparates bedient, gelöst.

Bevorzugte Ausführungsformen vorliegender Erfindung betreffen ein pharmazeutisches Kombinationspräparat der eingangs angegebenen Art, bei dem
- die erste Stufe 25 oder 26 tägliche Dosierungseinheiten umfaßt,
- die erste Stufe mindestens 28 und maximal 84 tägliche Dosierungseinheiten umfaßt,
- die erste Stufe 28 tägliche Dosierungseinheiten umfaßt.
- die erste Stufe 28 plus 7 oder 28 plus ein Mehrfaches von 7 tägliche Dosierungseinheiten umfaßt,
- die zweite Stufe 7 tägliche Dosierungseinheiten umfaßt.
- die erste Stufe 25 oder 26 tägliche Dosierungseinheiten und die zweite Stufe 5 oder 6 tägliche Dosierungseinheiten umfaßt, so daß das Kombinationspräparat insgesamt 30 oder 31 tägliche
   Dosierungseinheiten aufweist.

Die vorliegende Erfindung betrifft des weiteren ein kontrazeptionelles Kit enthaltend mindestens 30 und maximal 84 tägliche, jeweils mindestens einen hormonellen Wirkstoff enthaltende, Dosierungseinheiten mit einer ersten und einer zweiten Stufe, welches in seiner ersten Stufe als hormonellen Wirkstoff in Kombination ein Estrogen- und in mindestens zur Ovulationshemmung ausreichender Dosierung ein Gestagenpräparat in einstufiger Ausbildung und in der zweiten Stufe als hormonellen Wirkstoff lediglich ein Estrogenpräparat enthält, wobei die erste Stufe mindestens 25 und maximal 77 tägliche, diskret oder kontinuierlich applizierte Dosierungseinheiten und die zweite Stufe 5, 6 oder 7 tägliche, diskret oder kontinuierlich applizierte Dosierungseinheiten umfaßt und die Gesamtzahl der Tageseinheiten gleich der Gesamtzahl der Tage des gewünschten, mindestens30 und maximal 84 Tage langen, Zyklus ist.

Bevorzugte kontrazeptionelle Kits gemäß vorliegender Erfindung zeichnen sich dadurch aus, daß deren
- erste Stufe 25 oder 26 tägliche Dosierungseinheiten umfaßt,
- erste Stufe mindestens 28 und maximal 84 tägliche Dosierungseinheiten umfaßt,
- erste Stufe 28 tägliche Dosierungseinheiten umfaßt,
- erste Stufe 28 plus 7 oder 28 plus ein Mehrfaches von 7 täglichen Dosierungseinheiten umfaßt,
- zweite Stufe 7 tägliche Dosierungseinheiten umfaßt,
- erste Stufe 25 oder 26 tägliche Dosierungseinheiten und deren zweite Stufe 5 oder 6 tägliche Dosierungseinheiten umfaßt, so daß das Kit insgesamt 30 oder 31 tägliche Dosierungseinheiten aufweist.

In einer weiteren Ausführungsform des erfindungsgemäßen kontrazeptionellen Kits befinden sich die Dosierungseinheiten der ersten Stufe teilweise in sich periodisch wiederholenden Untereinheiten, die räumlich und/oder durch andere Markierungen voneinander getrennt sind.

Vorzugsweise befinden sich die Dosierungseinheiten frühestens ab der 26. täglichen Dosierungseinheit in Untereinheiten, lassen sich die einzelnen Untereinheiten durch Perforierungen oder andere zum Abtrennen geeignete Vorrichtungen voneinander trennen, sind in den separaten Untereinheiten jeweils 7 Dosierungseinheiten enthalten, umfaßt die erste Stufe 28 plus 7 oder 28 plus ein Mehrfaches von 7 täglichen Dosierungseinheiten und/oder die zweite Stufe 7 tägliche Dosierungseinheiten.

Bei der erfindungsgemäßem kontrazeptiven Methode, die sich des beschriebenen Kombinationspräparates bedient, wird in der ersten Stufe beginnend mit dem ersten Zyklustag eine Dosierungseinheit enthaltend ein Estrogen in Kombination mit einer gestagenen Komponente täglich über mindestens 25 und maximal 77 Tage verabreicht. Daran schliesst sich die zweite Stufe an, in der über den verbleibenden Zeitraum zum mindestens 30 und maximal 84 Tage umfassenden Zyklus über 5, 6 oder 7 Tage ein Estrogen verabreicht wird. Es wird also an jedem Tag des Zyklus eine einen hormonellen Wirkstoff enthaltende Dosierungseinheit verabreicht.

Bei Einnahme des erfindungsgemäßen Kombinationspräparates wird die Rekrutierung des dominanten Follikels, die im spontanen Zyklus während der ersten 6 Tage des Menstruationszyklus erfolgt, effizient unterdrückt. Somit lassen sich mit dem Kombinationspräparat der vorliegenden Erfindung die Follikelentwicklung unterdrücken und damit Durchbruchsovulationen vermeiden, wodurch die kontrazeptive Sicherheit erhöht wird.
Dies ist vor allem bei Einnahmefehlern von eminenter Wichtigkeit, und zwar insbesondere bei hormonalen Kontrazeptiva mit niedriger täglicher Ethinylestradiol-Dosismenge. Da bei 25% der Frauen, die die Pille nehmen, Einnahmefehler (Auslassen von Dosierungseinheiten oder Verlängerung des Intervalls auf über 24 Stunden zwischen der täglichen Einnahme zweier Dosierungseinheiten) bekannt sind (Finlay I.G., Scott M.B.G.: Patterns of contraceptive pill-taking in an inner city practice. Br.Med.J. 1986, 293: 601-602), erhöht das erfindungsgemäße Kombinatioinspräparat, wenn es als ovulationshemmendes Mittel verwendet wird, die kontrazeptive Sicherheit. Dies trifft insbesondere bei niedrigst dosierten Präparaten zu.

Insbesondere aber führt diese höhere Anzahl sowohl estrogen- als auch gestagenhaltiger täglicher Dosierungseinheiten zu einer Zyklusverlängerung und einer geringeren Häufigkeit von Abbruchblutungen.
Die anschließende Stufe, in der über 5, 6 oder 7 Tage Dosierungseinheiten täglich verabreicht werden, die nur eine estrogene Komponente als hormonalen Wirkstoff enthalten, gewährleistet eine Abbruchsblutung und bewirkt eine Stimulierung von Progesteronrezeptoren im Endometrium, wodurch im folgenden Verabreichungszyklus eine verringerte Zwischenblutungsrate verglichen mit herkömmlichen, niedrig dosierten Präparaten erreicht wird.

In Verbindung mit der verlängerten Phase der Verabreichung estrogen- und gestagenhaltiger Dosierungseinheiten wird so zum Beispiel eine Verlängerung der Menstruationszyklen auf 30 bzw. 31 Tage möglich (Monatspackung) möglich.

Gemäß der bevorzugten Varianten ist eine beliebiges Herausschieben der Abbruchblutung bis nach dem Tag 77 möglich, die nach dem Absetzen der estrogenund gestagenhaltiger Dosierungseinheiten einsetzt.

Eine variable Handhabung des Einleitens der Abbruchblutung ist mit dem kontrazeptionellen Kit gemäß vorliegender Erfindung möglich, bei dem sich die Dosierungseinheiten der ersten Stufe, frühestens ab der 26. täglichen Dosierungseinheit, in Untereinheiten befinden, welche sich aufgrund vorhandener Perforierungen oder anderer zum Abtrennen geeigneter Vorrichtungen voneinander trennen lassen.

Das erfindungsgemäße kontrazeptionelle Kit ist zum Beispiel in Form eines Blisters ausgebildet, bei welchem sich jeweils einzelne Sekmente, die vorzugsweise jeweils 7 Dosierungseinheiten der ersten Stufe enthalten, aufgrund von auf der Basisplatte des Blisters angebrachter Perforierungen bequem und einfach abtrennen lassen.
Es ist aber auch möglich, daß die Untereinheiten jeweils als eigene, separate Blister vorliegen oder die beispielsweise 28 Dosierungseinheiten der ersten Stufe in einem ersten Blister und die folgenden Dosierungseinheiten der ersten Stufe in einem zweiten Blister, bei dem sich in der beschriebenen Weise Untereinheiten durch Perforierungen abtrennen lassen.

Vorzugsweise wird in allen Ausführungsformen der Erfindung das Estrogen der ersten Hormonkomponente ausgewählt aus der Gruppe der Verbindungen
17β-Estradiol,
Ethinylestradiol und
17β-Estradiolvalerat
und das Gestagen ausgewählt aus der Gruppe der Verbindungen
Dienogest
Gestoden,
Levonorgestrel,
Desogestrel,
3-Ketodesogestrel,
Drospironenon,
Cyptoeronacetat,
Norgestimat und
Norethisteron sowie
das Estrogen der zweiten Hormonkomponente ausgewählt aus der Gruppe der Verbindungen
17β-Estradiol,
Ethinylestradiol und
17β-Estradiolvalerat.

Vorzugsweise ist in der vorliegenden Erfindung das Estrogen der ersten Hormonkomponente in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
1.0 bis 3.0 mg Dienogest
0.05 bis 0.075 mg Gestoden,
0.05 bis 0.125 mg Levonorgestrel,
0.06 bis 0.15 mg Desogestrel.
0.06 bis 0.15 mg 3-Ketodesogestrel,
1.0 bis 3.0 mg Drospironenon,
1.0 bis 2.0 mg Cyptoeronacetat,
0.2 mg bis 0.3 mg Norgestimat,
0.35 bis 0.75 mg Norethisteron
enthalten.

Die zweite Hormonkomponente enthält das Estrogen in jeder täglichen Dosierungseinheit vorzugsweise in einer Menge von
1.0 bis 6.0 mg 17β-Estradiol,
0.002 bis 0.04 mg Ethinylestradiol,
1.0 bis 4.0 mg 17β-Estradiolvalerat.

Gemäß einer besonders bevorzugten Ausführungsform enthält die zweite Hormonkomponente als Estrogen Ethinylestradiol in jeder täglichen Dosierungseinheit in einer Menge von 0.01 bis 0.015 mg.

Als Estrogen für die erste sowohl als auch die zweite Hormonkomponente kommt in erster Linie Ethinylestradiol oder das natürliche Estradiol in Betracht.

Von den genannten Gestagenen fiir die zweite Hormonkomponente ist Gestoden hervorzuheben; auch Levonorgestrel ist bevorzugt.

17β-Estradiolvalerat, welches als Estrogen sowohl in der ersten als auch in der zweiten Hormonkomponente enthalten sein kann, ist nur als ein möglicher Vertreter dieser 17β-Estradiolester genannt; auch andere derartige, homologe Ester können als estrogene Komponente im Rahmen der vorliegenden Erfindung verwendet werden.

Das nachfolgende Beispiel dient der näheren Erläuterung der vorliegenden Erfindung:

| **Beispiel 1:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | | | |
| Zusammensetzung | C | C | C | C | | | |
| | | | | | | | |
| Tag | 26 | 27 | 28 | 29 | 30 | | |
| Zusammensetzung | E | E | E | E | E | | |

| **Beispiel 2:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | 26 | | |
| Zusammensetzung | C | C | C | C | C | | |
| | | | | | | | |
| Tag | 27 | 28 | 29 | 30 | 31 | | |
| Zusammensetzung | E | E | E | E | E | | |

| **Beispiel 3:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | | | |
| Zusammensetzung | C | C | C | C | | | |
| | | | | | | | |
| Tag | 26 | 27 | 28 | 29 | 30 | 31 | |
| Zusammensetzung | E | E | E | E | E | E | |

| **Beispiel 4:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | 26 | 27 | 28 |
| Zusammensetzung | C | C | C | C | C | C | C |
| | | | | | | | |
| Tag | 29 | 30 | 31 | 32 | 33 | 34 | 35 |
| Zusammensetzung | C | C | C | C | C | C | C |
| | | | | | | | |
| Tag | 36 | 37 | 38 | 39 | 40 | 41 | 42 |
| Zusammensetzung | C | C | C | C | C | C | C |
| | | | | | | | |
| Tag | 43 | 44 | 45 | 46 | 47 | 48 | 49 |
| Zusammensetzung | C | C | C | C | C | C | C |
| | | | | | | | |
| Tag | 50 | 51 | 52 | 53 | 54 | 55 | 56 |
| Zusammensetzung | C | C | C | C | C | C | C |
| | | | | | | | |
| Tag | 57 | 58 | 59 | 60 | 61 | 62 | 63 |
| Zusammensetzung | E | E | E | E | E | E | E |

| **Beispiel 5:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | 26 | 27 | 28 |
| Zusammensetzung | C | C | C | C | C | C | C |
| | | | | | | | |
| Tag | 29 | 30 | 31 | 32 | 33 | 34 | 35 |
| Zusammensetzung | C | C | C | C | C | C | C |
| | | | | | | | |
| Tag | 36 | 37 | 38 | 39 | 40 | 41 | 42 |
| Zusammensetzung | C | C | C | C | C | C | C |
| | | | | | | | |
| Tag | 43 | 44 | 45 | 46 | 47 | 48 | 49 |
| Zusammensetzung | C | C | C | C | C | C | C |
| | | | | | | | |
| Tag | 50 | 51 | 52 | 53 | 54 | 55 | 56 |
| Zusammensetzung | C | C | C | C | C | C | C |
| | | | | | | | |
| Tag | 57 | 58 | 59 | 60 | 61 | 62 | 63 |
| Zusammensetzung | C | C | C | C | C | C | C |
| Tag | 64 | 65 | 66 | 67 | 68 | 69 | 70 |
| Zusammensetzung | C | C | C | C | C | C | C |
| | | | | | | | |
| Tag | 71 | 72 | 73 | 74 | 75 | 76 | 77 |
| Zusammensetzung | C | C | C | C | C | C | C |
| | | | | | | | |
| Tag | 78 | 79 | 80 | 81 | 82 | 83 | 84 |
| Zusammensetzung | E | E | E | E | E | E | E |

| **Beispiel 6:** | | | | | | | |
|---|---|---|---|---|---|---|---|
| Tag | 1 | 2 | 3 | 4 | 5 | 6 | 7 |
| Zusammensetzung | C | C | C | C | C | C | C |
| | | | | | | | |
| Tag | 8 | 9 | 10 | 11 | 12 | 13 | 14 |
| Zusammensetzung | C | C | C | C | C | C | C |
| | | | | | | | |
| Tag | 15 | 16 | 17 | 18 | 19 | 20 | 21 |
| Zusammensetzung | C | C | C | C | C | C | C |
| | | | | | | | |
| Tag | 22 | 23 | 24 | 25 | 26 | 27 | 28 |
| Zusammensetzung | C | C | C | C | C | C | C |
| | | | | | | | |
| Tag | 29 | 30 | 31 | 32 | 33 | 34 | 35 |
| Zusammensetzung | C | C | C | C | C | C | C |
| | | | | | | | |
| Tag | 36 | 37 | 38 | 39 | 40 | 41 | 42 |
| Zusammensetzung | C | C | C | C | C | C | C |
| | | | | | | | |
| Tag | 43 | 44 | 45 | 46 | 47 | 48 | 49 |
| Zusammensetzung | C | C | C | C | C | C | C |
| | | | | | | | |
| Tag | 50 | 51 | 52 | 53 | 54 | 55 | 56 |
| Zusammensetzung | E | E | E | E | E | E | E |
| Tag = Tag des Menstruationszyklus. Tag 1 ist der erste Blutungstag | | | | | | | |
| C = Kombination von Estrogen und Gestagen (= erste Hormonkomponente) | | | | | | | |
| E = Estrogen (= zweite Hormonkomponente), P = Perforierung | | | | | | | |

Die Formulierung der Dosierungseinheiten erfolgt konventionell unter Verwendung von für die Herstellung Estrogen-/Gestagen- sowie ausschließlich Estrogen-haltiger Tabletten, Pillen, Dragees, etc. bekannter Hilfsstoffe.

Das erfindungsgemäße Kombinationspräparat dient der Empfängnisverhütung für die Frau durch Verabreichung der täglichen Dosierungseinheiten der ersten Hormonkomponente über mindestens 25 und maximal 77 Tage, beginnend am Tag eins des Menstruationszyklus (erster Tag der Menstruationsblutung), gefolgt von 5, 6 oder 7 täglichen Dosierungseinheiten der zweiten Hormonkomponente, die ausschließlich ein Estrogen (E) enthalten, während insgesamt mindestens 30 und maximal 84 Tagen im Verabreichungszyklus. Im zweiten (nächsten) Menstruationszyklus wird an dessen Tag eins ohne Einnahmepause mit der Einnahme der ersten Dosierungseinheit der ersten Hormonkomponente einer neuen Verpackungseinheit (Kit) des erfindungsgemäßen pharmazeutischen Kombinationspräparates weiterverfahren.

Mit diesem Kombinationspräparat kann eine ausgeprägte ovarielle Suppression ohne häufige Follikelanreifung sowie hervorragender Zykluskontrolle bei niedriger täglicher Estrogendosierung, niedriger Gesamtestrogen- sowie niedriger Gesamthormonmenge pro Verabreichungszyklus erreicht werden.

Die Vorteile dieses über mindestens 30 und höchstens 84 Tage pro Zyklus verabreichten, erfindungsgemäßen Kombinationspräparates (ovulationshemmendes Mittel) gegenüber den bisher beschriebenen Präparaten, insbesondere denen mit einer täglichen Ethinylestradiol-Dosis von weniger als 30µg und solchen mit pillenfreiem Intervall, lassen sich wie folgt charakterisieren:
1. Eine signifikant geringere Häufigkeit von Follikelentwicklungen bei der Anwenderin Dies bedeutet eine geringere Gefahr von Durchbruchovulationen und damit eine grössere kontrazeptive Zuverlässigkeit insbesondere bei Einnahmefehlern.
2. Die Rekrutierung des dominanten Follikels wird durch die Verlängerung der Einnahme der Kombination wirksam unterdrückt.
3. Die Einnahme von 5, 6 oder 7 täglichen Estrogen-Dosierungseinheiten im Anschluss an die Verabreichung der Kombinationsdosierung der ersten Stufe führt zu einer deutlich verbesserten Zykluskontrolle und zu einer geringeren Inzidenz von Nebenwirkungen wie Kopfschmerzen im Rahmen des prämenstruellen Syndroms.
4. Andere klinische Symptome, die auf stark fluktuierende endogene Estrogenspiegel zurückzuführen sind, wie beispielsweise Brustspannen, sind aufgrund der bedeutend stärkeren ovariellen Supprimierung ebenfalls deutlich verringert.
5. Durch die Blutungsfreiheit (Amenorrhoe) über einen längeren Zeitraum erhöht sich die Akzeptanz durch die Anwenderin; dies gilt insbesondere mit steigender Zahl der Dosierungseinheiten in der ersten Stufe.
6. Die mit bzw. durch die Blutungen eintretenden negativen Effekte, wie z.B. Anämie. Krämpfe, Unwohlsein etc. treten seltener auf.
7. Konstante niedrige Estrogen- und Gestagenspiegel; dadurch Vermeidung metabolischer Schwankungen und damit einhergehende Verbesserung der Verträglichkeit.
8. Eine eventuelle Schwangerschaft aufgrund von Einnahmefehlern wird durch die nach dem Absetzen der estrogen- und gestagenhaltigen Dosierungseinheiten dann ausbleibende Abbruchblutung erkennbar.

Die Formulierung eines Estrogens und Gestagens für die Herstellung eines erfindungsgemäßen Kombinationspräparates erfolgt vollkommen analog wie es bereits für herkömmliche orale Kontrazeptiva mit 21tägiger Einnahmedauer der Wirkstoffe. wie beispielsweise Femovan® (Ethinylestradiol/Gestoden) oder Microgynon® (Ethinylestradiol/Levonorgestrel) bekannt ist. Die Formulierung der ausschließlich Estrogen-haltigen Dosierungseinheiten kann ebenso ganz analog wie es für schon erhältliche, zur oralen Anwendung bestimmten Estrogen-haltige Mittel, beispielsweise ProgynonC®, bekannt ist, durchgeführt werden.
Die Dosierungseinheiten der ersten Stufe des erfindungsgemäßen Präparates können auch in Form eines Pflasters (transdermale Applikation), eines Implants oder einer anderen Depotformulierung ausgebildet sein und so kontinuierlich appliziert werden.

Als Verpackungsform fiir das erfindungsgemäße Kombinationspräparat dient im allgemeinen eine Blisterpackung, die in der erfindungsgemäßen Art ausgebildet ist; jedoch sind auch andere fiir diesen Zweck bekannte Verpackungsformen denkbar.

Zur Bestimmung wirkequivalenter Mengen von Ethinylestradiol und 17β-Estradiol einerseits und verschiedener Gestagene wie Gestoden, Levonorgestrel, Desogestrel und 3-Ketodesogestrel andererseits wird auf die in der EP-A- 0 253 607 gemachten Angaben verwiesen. Weitere Einzelheiten zur Bestimmung von Dosisequivalenten verschiedener gestagener Wirkstoffe finden sich beispielsweise in "Probleme der Dosisfindung: Sexualhormone"; F. Neumann et al. in "Arzneimittelforschung" (Drug Research) 27, 2a, 296 - 318 (1977) sowie in "Aktuelle Entwicklungen in der hormonalen Kontrazeption"; H. Kühl in "Gynäkologe" 25: 231 - 240 (1992).

## Patentansprüche

1. Zweistufiges pharmazeutisches Kombinationspräparat zur hormonalen Kontrazeption enthaltend mindestens 30 tägliche Dosierungseinheiten, welches in seiner ersten Stufe als hormonellen Wirkstoff in Kombination ein Estrogen- und in mindestens zur Ovulationshemmung ausreichender Dosierung ein Gestagenpräparat in einstufiger Ausbildung und in der zweiten Stufe als hormonellen Wirkstoff lediglich ein Estrogenpräparat enthält, wobei die erste Stufe mindestens 25 und maximal 77 tägliche, diskret oder kontinuierlich applizierte Dosierungseinheiten und die zweite Stufe 5, 6 oder 7 tägliche, diskret oder kontinuierlich applizierte Dosierungseinheiten umfaßt und die Gesamtzahl der Tageseinheiten gleich der Gesamtzahl der Tage des gewünschten, mindestens30 und maximal 84 Tage langen, Zyklus ist,

2. Kombinationspräparat nach Anspruch 1, **dadurch gekennzeichnet, daß** die erste Stufe 25 oder 26 tägliche Dosierungseinheiten umfaßt.

3. Kombinationspräparat nach Anspruch 1, **dadurch gekennzeichnet, daß** die erste Stufe mindestens 28 und maximal 84 tägliche Dosierungseinheiten umfaßt.

4. Kombinationspräparat nach Anspruch 3, **dadurch gekennzeichnet, daß** die erste Stufe 28 tägliche Dosierungseinheiten umfaßt.

5. Kombinationspräparat nach Anspruch 3, **dadurch gekennzeichnet, daß** die erste Stufe 28 plus 7 oder 28 plus ein Mehrfaches von 7 tägliche Dosierungseinheiten umfaßt.

6. Kombinationspräparat nach Anspruch 1, **dadurch gekennzeichnet, daß** die zweite Stufe 7 tägliche Dosierungseinheiten umfaßt.

7. Kombinationspräparat nach Anspruch 2, **dadurch gekennzeichnet, daß** die zweite Stufe 5 oder 6 tägliche Dosierungseinheiten umfaßt, so daß das Kombinationspräparat insgesamt 30 oder 31 tägliche Dosierungseinheiten aufweist.

8. Kombinationspräparat nach einem der vorstehenden Ansprüche 1 bis 7, **dadurch gekennzeichnet, daß** das Estrogen der ersten Stufe aus der Gruppe der Verbindungen
Ethinylestradiol und
17β-Estradiolvalerat
und das Gestagen aus der Gruppe der Verbindungen
Dienogest
Gestoden,
Levonorgestrel,
Desogestrel,
3-Ketodesogestrel,
Drospironenon,
Cyptoeronacetat,
Norgestimat und
Norethisteron sowie
das Estrogen der zweiten Hormonkomponente aus der Gruppe der Verbindungen.
17β-Estradiol,
Ethinylestradiol und
17β-Estradiolvalerat
ausgewählt ist.

9. Kombinationspräparat nach einem der vorstehenden Ansprüche 1 bis 8, **dadurch gekennzeichnet, daß** das Estrogen der ersten Stufe in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
1.0 bis 3.0 mg Dienogest
0.05 bis 0.075 mg Gestoden
0.05 bis 0.125 mg Levonorgestrel
0.06 bis 0.15 mg Desogestrel
0.06 bis 0.15 mg 3-Ketodesogestrel
1.0 bis 3.0 mg Drospironenon
1.0 bis 2.0 mg Cyptoeronacetat
0.2 mg bis 0.3 mg Norgestimat
0.35 bis 0.75 mg Norethisteron
enthalten ist.

10. Kombinationspräparat nach einem der vorstehenden Ansprüche 1 bis 9, **dadurch gekennzeichnet, daß** in der zweiten Stufe in jeder täglichen Dosierungseinheit eine Menge von
1.0 bis 6.0 mg 17β-Estradiol,
0.002 bis 0.04 mg Ethinylestradiol,
1.0 bis 4.0 mg 17β-Estradiolvalerat enthalten ist.

11. Kontrazeptionelles Kit enthaltend mindestens 30 tägliche, jeweils mindestens einen hormonellen Wirkstoff enthaltende, Dosierungseinheiten mit einer ersten und einer zweiten Stufe,
welches in seiner ersten Stufe als hormonellen Wirkstoff in Kombination ein Estrogen- und in mindestens zur Ovulationshemmung ausreichender Dosierung ein Gestagenpräparat in einstufiger Ausbildung und in der zweiten Stufe als hormonellen Wirkstoff lediglich ein Estrogenpräparat enthält, wobei die erste Stufe mindestens 25 und maximal 77 tägliche, diskret oder kontinuierlich applizierte Dosierungseinheiten und die zweite Stufe 5, 6 oder 7 tägliche, diskret oder kontinuierlich applizierte Dosierungseinheiten umfaßt und die Gesamtzahl der Tageseinheiten gleich der Gesamtzahl der Tage des gewünschten, mindestens30 und maximal 84 Tage langen, Zyklus ist.

12. Kontrazeptionelles Kit nach Anspruch 11, **dadurch gekennzeichnet, daß** dessen erste Stufe 25 oder 26 tägliche Dosierungseinheiten umfaßt.

13. Kontrazeptionelles Kit nach Anspruch 11, **dadurch gekennzeichnet, daß** die erste Stufe mindestens 28 und maximal 84 tägliche Dosierungseinheiten umfaßt.

14. Kontrazeptionelles Kit nach Anspruch 13, **dadurch gekennzeichnet, daß** die erste Stufe 28 tägliche Dosierungseinheiten umfaßt.

15. Kontrazeptionelles Kit nach Anspruch 13, **dadurch gekennzeichnet, daß** die erste Stufe 28 plus 7 oder 28 plus ein Mehrfaches von 7 tägliche Dosierungseinheiten umfaßt.

16. Kontrazeptionelles Kit nach Anspruch 11, **dadurch gekennzeichnet, daß** die zweite Stufe 7 tägliche Dosierungseinheiten umfaßt.

17. Kontrazeptionelles Kit nach Anspruch 12, **dadurch gekennzeichnet, daß** die zweite Stufe 5 oder 6 tägliche Dosierungseinheiten umfaßt, so daß das Kit insgesamt 30 oder 31 tägliche Dosierungseinheiten aufweist.

18. Kontrazeptionelles Kit nach einem der vorstehenden Ansprüche 11 bis 17, **dadurch gekennzeichnet, daß** das Estrogen der ersten Stufe aus der Gruppe der Verbindungen
17β-Estradiol,
Ethinylestradiol und
17β-Estradiolvalerat
und das Gestagen aus der Gruppe der Verbindungen
Dienogest
Gestoden,
Levonorgestrel,
Desogestrel,
3-Ketodesogestrel,
Drospironenon,
Cyptoeronacetat,
Norgestimat und
Norethisteron sowie
das Estrogen der zweiten Hormonkomponente aus der Gruppe der Verbindungen.
17β-Estradiol,
Ethinylestradiol und
17β-Estradiolvalerat
ausgewählt ist.

19. Kontrazeptionelles Kit nach einem der vorstehenden Ansprüche 11 bis 18, **dadurch gekennzeichnet, daß** das Estrogen der ersten Stufe in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
1.0 bis 3.0 mg Dienogest
0.05 bis 0.075 mg Gestoden
0.05 bis 0.125 mg Levonorgestrel
0.06 bis 0.15 mg Desogestrel
0.06 bis 0.15 mg 3-Ketodesogestrel
1.0 bis 3.0 mg Drospironenon
1.0 bis 2.0 mg Cyptoeronacetat
0.2 mg bis 0.3 mg Norgestimat
0.35 bis 0.75 mg Norethisteron
enthalten ist.

20. Kontrazeptionelles Kit nach einem der vorstehenden Ansprüche 11 bis 19, **dadurch gekennzeichnet, daß** in der zweiten Stufe in jeder täglichen Dosierungseinheit eine Menge von
1.0 bis 6.0 mg 17β-Estradiol,
0.002 bis 0.04 mg Ethinylestradiol,
1.0 bis 4.0 mg 17β-Estradiolvalerat
enthalten ist.

21. Kontrazeptionelles Kit enthaltend mindestens 30 tägliche, jeweils mindestens einen hormonellen Wirkstoff enthaltende, Dosierungseinheiten mit einer ersten und einer zweiten Stufe,
welches in seiner ersten Stufe als hormonellen Wirkstoff in Kombination ein Estrogen- und in mindestens zur Ovulationshemmung ausreichender Dosierung ein Gestagenpräparat in einstufiger Ausbildung und in seiner zweiten Stufe als hormonellen Wirkstoff lediglich ein Estrogenpräparat enthält, wobei die erste Stufe mindestens 25 und maximal 77 tägliche, diskret oder kontinuierlich applizierte Dosierungseinheiten und die zweite Stufe 5, 6 oder 7 tägliche, diskret oder kontinuierlich applizierte Dosierungseinheiten umfaßt und die Gesamtzahl der Tageseinheiten gleich der Gesamtzahl der Tage des gewünschten, mindestens 30 und maximal 84 Tage langen, Zyklus ist,
wobei sich die Dosierungseinheiten der ersten Stufe teilweise in sich periodisch wiederholenden Untereinheiten befinden, die räumlich und/oder durch andere Markierungen voneinander getrennt sind.

22. Kontrazeptionelles Kit nach Anspruch 21, **dadurch gekennzeichnet, daß** sich die Dosierungseinheiten frühestens ab der 26. täglichen Dosierungseinheit in Untereinheiten befinden.

23. Kontrazeptionelles Kit nach Anspruch 21 oder 22, **dadurch gekennzeichnet, daß** sich die einzelnen Untereinheiten durch Perforierungen oder andere zum Abtrennen geeignete Vorrichtungen voneinander trennen lassen.

24. Kontrazeptionelles Kit nach einem der Ansprüche 21, 22 oder 23, **dadurch gekennzeichnet, daß** die separaten Untereinheiten jeweils 7 Dosierungseinheiten enthalten.

25. Kontrazeptionelles Kit nach einem der vorstehenden Ansprüche 21 bis 24, **dadurch gekennzeichnet, daß** die erste Stufe 28 plus 7 oder 28 plus ein Mehrfaches von 7 tägliche Dosierungseinheiten umfaßt.

26. Kontrazeptionelles Kit nach einem der vorstehenden Ansprüche 21 bis 25, **dadurch gekennzeichnet, daß** die zweite Stufe 7 tägliche Dosierungseinheiten umfaßt.

27. Kontrazeptionelles Kit nach einem der vorstehenden Ansprüche 21 bis 26, **dadurch gekennzeichnet, daß** das Estrogen der ersten Stufe aus der Gruppe der Verbindungen
17β-Estradiol,
Ethinylestradiol und
17β-Estradiolvalerat
und das Gestagen aus der Gruppe der Verbindungen
Dienogest
Gestoden,
Levonorgestrel,
Desogestrel,
3-Ketodesogestrel,
Drospironenon,
Cyptoeronacetat,
Norgestimat und
Norethisteron sowie
das Estrogen der zweiten Hormonkomponente aus der Gruppe der Verbindungen.
17β-Estradiol,
Ethinylestradiol und
17β-Estradiolvalerat
ausgewählt ist.

28. Kontrazeptionelles Kit nach einem der vorstehenden Ansprüche 21 bis 27, **dadurch gekennzeichnet, daß** das Estrogen der ersten Stufe in jeder täglichen Dosierungseinheit in einer Dosis von
1.0 bis 6.0 mg 17β-Estradiol,
0.015 bis 0.025 mg Ethinylestradiol,
1.0 bis 4.0 mg 17β-Estradiolvalerat
und das Gestagen in jeder täglichen Dosierungseinheit in einer Dosis von
1.0 bis 3.0 mg Dienogest
0.05 bis 0.075 mg Gestoden
0.05 bis 0.125 mg Levonorgestrel
0.06 bis 0.15 mg Desogestrel
0.06 bis 0.15 mg 3-Ketodesogestrel
1.0 bis 3.0 mg Drospironenon
1.0 bis 2.0 mg Cyptoeronacetat
0.2 mg bis 0.3 mg Norgestimat
0.35 bis 0.75 mg Norethisteron
enthalten ist.

29. Kontrazeptionelles Kit nach einem der vorstehenden Ansprüche 21 bis 28, **dadurch gekennzeichnet, daß** in der zweiten Stufe in jeder täglichen Dosierungseinheit eine Menge von
1.0 bis 6.0 mg 17β-Estradiol,
0.002 bis 0.04 mg Ethinylestradiol,
1.0 bis 4.0 mg 17β-Estradiolvalerat
enthalten ist.

## Claims

1. Two-stage pharmaceutical combined preparation for hormonal contraception containing at least 30 daily unit doses, which preparation, in its first stage, comprises as hormonal active ingredient a combination of an oestrogen preparation and, in a dose that is at least sufficient to inhibit ovulation, a gestagen preparation, in single-stage form and, in the second stage, comprises as hormonal active ingredient an oestrogen preparation only, wherein the first stage comprises a minimum of 25 and a maximum of 77 daily discrete or continuous unit doses and the second stage comprises 5, 6 or 7 daily discrete or continuous unit doses, and wherein the total number of daily units is equal to the total number of days of the desired cycle of a minimum of 30 and a maximum of 84 days.

2. Combined preparation according to claim 1, **characterized in that** the first stage comprises 25 or 26 daily unit doses.

3. Combined preparation according to claim 1, **characterized in that** the first stage comprises a minimum of 28 and a maximum of 84 daily unit doses.

4. Combined preparation according to claim 3, **characterized in that** the first stage comprises 28 daily unit doses.

5. Combined preparation according to claim 3, **characterized in that** the first stage comprises 28 plus 7, or 28 plus a multiple of 7, daily unit doses.

6. Combined preparation according to claim 1, **characterized in that** the second stage comprises 7 daily unit doses.

7. Combined preparation according to claim 2, **characterized in that** the second stage comprises 5 or 6 daily unit doses, so that the combined preparation has a total of 30 or 31 daily unit doses.

8. Combined preparation according to one of the preceding claims 1 to 7, **characterized in that** the oestrogen of the first stage is selected from the group of compounds
17β-oestradiol,
ethynyloestradiol and
17β-oestradiol valerate
and the gestagen is selected from the group of compounds
dienogest,
gestodene,
levonorgestrel,
desogestrel,
3-ketodesogestrel,
drospironenone,
cyproterone acetate,
norgestimate and
norethisterone and also
the oestrogen of the second hormone component is selected from the group of compounds
17β-oestradiol,
ethynyloestradiol and
17β-oestradiol valerate.

9. Combined preparation according to one of the preceding claims 1 to 8, **characterized in that** the oestrogen of the first stage is contained in each daily unit dose in a dose of
from 1.0 to 6.0 mg of 17β-oestradiol,
from 0.015 to 0.025 mg of ethynyloestradiol,
from 1.0 to 4.0 mg of 17β-oestradiol valerate
and the gestagen is contained in each daily unit dose in a dose of
from 1.0 to 3.0 mg of dienogest,
from 0.05 to 0.075 mg of gestodene,
from 0.05 to 0.125 mg of levonorgestrel,
from 0.06 to 0.15 mg of desogestrel,
from 0.06 to 0.15 mg of 3-ketodesogestrel,
from 1.0 to 3.0 mg of drospironenone,
from 1.0 to 2.0 mg of cyproterone acetate,
from 0.2 mg to 0.3 mg of norgestimate
from 0.35 to 0.75 mg of norethisterone.

10. Combined preparation according to one of the preceding claims 1 to 9, **characterized in that** there is contained in each daily unit dose in the second stage an amount of
from 1.0 to 6.0 mg of 17β-oestradiol,
from 0.002 to 0.04 mg of ethynyloestradiol,
from 1.0 to 4.0 mg of 17β-oestradiol valerate.

11. Contraceptive kit containing at least 30 daily unit doses each comprising at least one hormonal active ingredient, having a first and a second stage, which kit, in its first stage, comprises as hormonal active ingredient a combination of an oestrogen preparation and, in a dose that is at least sufficient to inhibit ovulation, a gestagen preparation, in single-stage form and, in the second stage, comprises as hormonal active ingredient an oestrogen preparation only, wherein the first stage comprises a minimum of 25 and a maximum of 77 daily discrete or continuous unit doses and the second stage comprises 5, 6 or 7 daily discrete or continuous unit doses, and wherein the total number of daily units is equal to the total number of days of the desired cycle of a minimum of 30 and a maximum of 84 days.

12. Contraceptive kit according to claim 11, **characterized in that** the first stage comprises 25 or 26 daily unit doses.

13. Contraceptive kit according to claim 11, **characterized in that** the first stage comprises a minimum of 28 and a maximum of 84 daily unit doses.

14. Contraceptive kit according to claim 13, **characterized in that** the first stage comprises 28 daily unit doses.

15. Contraceptive kit according to claim 13, **characterized in that** the first stage comprises 28 plus 7, or 28 plus a multiple of 7, daily unit doses.

16. Contraceptive kit according to claim 11, **characterized in that** the second stage comprises 7 daily unit doses.

17. Contraceptive kit according to claim 12, **characterized in that** the second stage comprises 5 or 6 daily unit doses, so that the kit has a total of 30 or 31 daily unit doses.

18. Contraceptive kit according to one of the preceding claims 11 to 17, **characterized in that** the oestrogen of the first stage is selected from the group of compounds
17β-oestradiol,
ethynyloestradiol and
17β-oestradiol valerate
and the gestagen is selected from the group of compounds
dienogest,
gestodene,
levonorgestrel,
desogestrel,
3-ketodesogestrel,
drospironenone,
cyproterone acetate,
norgestimate and
norethisterone and also
the oestrogen of the second hormone component is selected from the group of compounds
17β-oestradiol,
ethynyloestradiol and
17β-oestradiol valerate.

19. Contraceptive kit according to one of the preceding claims 11 to 18, **characterized in that** the oestrogen of the first stage is contained in each daily unit dose in a dose of
from 1.0 to 6.0 mg of 17β-oestradiol,
from 0.015 to 0.025 mg of ethynyloestradiol,
from 1.0 to 4.0 mg of 17β-oestradiol valerate
and the gestagen is contained in each daily unit dose in a dose of
from 1.0 to 3.0 mg of dienogest,
from 0.05 to 0.075 mg of gestodene,
from 0.05 to 0.125 mg of levonorgestrel,
from 0.06 to 0.15 mg of desogestrel,
from 0.06 to 0.15 mg of 3-ketodesogestrel,
from 1.0 to 3.0 mg of drospironenone,
from 1.0 to 2.0 mg of cyproterone acetate,
from 0.2 mg to 0.3 mg of norgestimate
from 0.35 to 0.75 mg of norethisterone.

20. Contraceptive kit according to one of the preceding claims 11 to 19, **characterized in that** there is contained in each daily unit dose in the second stage an amount of
from 1.0 to 6.0 mg of 17β-oestradiol,
from 0.002 to 0.04 mg of ethynyloestradiol,
from 1.0 to 4.0 mg of 17β-oestradiol valerate.

21. Contraceptive kit containing at least 30 daily unit doses each comprising at least one hormonal active ingredient, having a first and a second stage, which kit, in its first stage, comprises as hormonal active ingredient a combination of an oestrogen preparation and, in a dose that is at least sufficient to inhibit ovulation, a gestagen preparation, in single-stage form and, in its second stage, comprises as hormonal active ingredient an oestrogen preparation only, wherein the first stage comprises a minimum of 25 and a maximum of 77 daily discrete or continuous unit doses and the second stage comprises 5, 6 or 7 daily discrete or continuous unit doses, and wherein the total number of daily units is equal to the total number of days of the desired cycle of a minimum of 30 and maximum of 84 days, some of the unit doses of the first stage being arranged in periodically repeating sub-units that are separated from one another spatially and/or by other markings.

22. Contraceptive kit according to claim 21, **characterized in that** the unit doses are arranged in sub-units at the earliest from the 26th daily unit dose.

23. Contraceptive kit according to claim 21 or 22, **characterized in that** the individual sub-units can be separated from one another by perforations or other means suitable for separation.

24. Contraceptive kit according to one of claims 21, 22 or 23, **characterized in that** the separate sub-units each contain 7 unit doses.

25. Contraceptive kit according to one of the preceding claims 21 to 24, **characterized in that** the first stage comprises 28 plus 7, or 28 plus a multiple of 7, daily unit doses.

26. Contraceptive kit according to one of the preceding claims 21 to 25, **characterized in that** the second stage comprises 7 daily unit doses.

27. Contraceptive kit according to one of the preceding claims 21 to 26, **characterized in that** the oestrogen of the first stage is selected from the group of compounds
17β-oestradiol,
ethynyloestradiol and
17β-oestradiol valerate
and the gestagen is selected from the group of compounds
dienogest,
gestodene,
levonorgestrel,
desogestrel,
3-ketodesogestrel,
drospironenone,
cyproterone acetate,
norgestimate and
norethisterone and also
the oestrogen of the second hormone component is selected from the group of compounds
17β-oestradiol,
ethynyloestradiol and
17β-oestradiol valerate.

28. Contraceptive kit according to one of the preceding claims 21 to 27, **characterized in that** the oestrogen of the first stage is contained in each daily unit dose in a dose of
from 1.0 to 6.0 mg of 17β-oestradiol,
from 0.015 to 0.025 mg of ethynyloestradiol,
from 1.0 to 4.0 mg of 17β-oestradiol valerate
and the gestagen is contained in each daily unit dose in a dose of
from 1.0 to 3.0 mg of dienogest,
from 0.05 to 0.075 mg of gestodene,
from 0.05 to 0.125 mg of levonorgestrel,
from 0.06 to 0.15 mg of desogestrel,
from 0.06 to 0.15 mg of 3-ketodesogestrel,
from 1.0 to 3.0 mg of drospironenone,
from 1.0 to 2.0 mg of cyproterone acetate,
from 0.2 mg to 0.3 mg of norgestimate
from 0.35 to 0.75 mg of norethisterone.

29. Contraceptive kit according to one of the preceding claims 21 to 28, **characterized in that** there is contained in each daily unit dose in the second stage an amount of
from 1.0 to 6.0 mg of 17β-oestradiol,
from 0.002 to 0.04 mg of ethynyloestradiol,
from 1.0 to 4.0 mg of 17β-oestradiol valerate.

## Revendications

1. Préparation pharmaceutique biphasique constituée d'une association de substances actives, pour la contraception hormonale, contenant au moins 30 doses unitaires journalières, qui contient dans sa première phase, en tant que substance active hormonale, en association, une préparation d'oestrogène et d'un progestatif à une dose au moins suffisante pour l'inhibition de l'ovulation, en configuration monophasique, et dans la seconde phase, en tant que substance active hormonale, seulement une préparation d'oestrogène, la première phase comprenant au moins 25 et au maximum 77 doses unitaires journalières, administrées en mode continu du discontinu, et la seconde phase comprenant 5, 6 ou 7 doses unitaires journalières administrées en mode continu ou discontinu, et le nombre total des doses unitaires étant égal au nombre total des jours du cycle désiré, d'une longueur d'au moins 30 et d'au maximum 84 jours.

2. Préparation constituée d'une association de substances actives selon la revendication 1, **caractérisée en ce que** la première phase comprend 25 ou 26 doses unitaires journalières.

3. Préparation constituée d'une association de substances actives selon la revendication 1, **caractérisée en ce que** la première phase comprend au moins 28 et au maximum 84 doses unitaires journalières.

4. Préparation constituée d'une association de substances actives selon la revendication 3, **caractérisée en ce que** la première phase comprend 28 doses unitaires journalières.

5. Préparation constituée d'une association de substances actives selon la revendication 3, **caractérisée en ce que** la première phase comprend 28 plus 7 ou 28 plus un multiple de 7 doses unitaires journalières.

6. Préparation constituée d'une association de substances actives selon la revendication 1, **caractérisée en ce que** la seconde phase comprend 7 doses unitaires journalières.

7. Préparation constituée d'une association de substances actives selon la revendication 2, **caractérisée en ce que** la seconde phase comprend 5 ou 6 doses unitaires journalières, de sorte que la préparation constituée d'une association de substances actives comporte au total 30 ou 31 doses unitaires journalières.

8. Préparation constituée d'une association de substances actives selon l'une quelconque des revendications précédentes 1 à 7, **caractérisée en ce que** l'oestrogène de la première phase est choisi dans le groupe des composés
17β-estradiol,
éthinylestradiol et
17β-estradiot valérate
et le progestatif est choisi dans le groupe des composés
diénogest,
gestodène,
lévonorgestrel,
désogestrel,
3-cétodésogestrel,
drospironénone,
cyprotérone acétate,
norgestimate et
noréthistérone, ainsi que
l'oestrogène du second composant hormonal est choisi dans le groupe des composés
17β-estradiol,
éthinylestradiol et
17β-estradiol valérate.

9. Préparation constituée d'une association de substances actives selon l'une quelconque des revendications précédentes 1 à 8, **caractérisée en ce que** l'oestrogène de la première phase est contenu dans chaque dose unitaire journalière à une dose de
1,0 à 6,0 mg de 17β-estradiol,
0,015 à 0,025 mg d'éthinylestradiol,
1,0 à 4,0 mg de 17β-estradiol valérate
et le progestatif est contenu dans chaque dose unitaire journalière à une dose de
1,0 à 3,0 mg de diénogest,
0,05 à 0,075 mg de gestodène,
0,05 à 0,125 mg de lévonorgestrel,
0,06 à 0,15 mg de désogestrel,
0,06 à 0,15 mg de 3-cétodésogestrel,
1,0 à 3,0 mg de drospironénone,
1,0 à 2,0 mg de cyprotérone acétate,
0,2 mg à 0,3 mg de norgestimate,
0,35 à 0,75 mg de noréthistérone.

10. Préparation constituée d'une association de substances actives selon l'une quelconque des revendications précédentes 1 à 9, **caractérisée en ce que** dans la seconde phase est contenue dans chaque dose unitaire journalière une quantité de
1,0 à 6,0 mg de 17β-estradiol,
0,002 à 0,04 mg d'éthinylestradiol,
1,0 à 4,0 mg de 17β-estradiol valérate.

11. Nécessaire de contraception contenant au moins 30 doses unitaires journalières contenant chacune au moins une substance active hormonale avec une première phase et une seconde,
qui contient dans sa première phase, en tant que substance active hormonale, en association, une préparation d'oestrogène et d'un progestatif à une dose au moins suffisante pour l'inhibition de l'ovulation, en configuration monophasique, et dans la seconde phase, en tant que substance active hormonale, seulement une préparation d'oestrogène, la première phase comprenant au moins 25 et au maximum 77 doses unitaires journalières, administrées en mode continu ou discontinu, et la seconde phase comprenant 5, 6 ou 7 doses unitaires journalières administrées en mode continu ou discontinu, et le nombre total des doses unitaires étant égal au nombre total des jours du cycle désiré, d'une longueur d'au moins 30 et d'au maximum 84 jours.

12. Nécessaire de contraception selon la revendication 11, **caractérisé en ce que** sa première phase comprend 25 ou 26 doses unitaires journalières.

13. Nécessaire de contraception selon la revendication 11, **caractérisé en ce que** la première phase comprend au moins 28 et au maximum 84 doses unitaires journalières.

14. Nécessaire de contraception selon la revendication 13, **caractérisé en ce que** la première phase comprend 28 doses unitaires journalières.

15. Nécessaire de contraception selon la revendication 13, **caractérisé en ce que** la première phase comprend 28 plus 7 ou 28 plus un multiple de 7 doses unitaires journalières.

16. Nécessaire de contraception selon la revendication 11, **caractérisé en ce que** la seconde phase comprend 7 doses unitaires journalières.

17. Nécessaire de contraception selon la revendication 12, **caractérisé en ce que** la seconde phase comprend 5 ou 6 doses unitaires journalières, de sorte que le nécessaire comporte au total 30 ou 31 doses unitaires journalières.

18. Nécessaire de contraception selon l'une quelconque des revendications précédentes 11 à 17, **caractérisé en ce que** l'oestrogène de la première phase est choisi dans le groupe des composés
17β-estradiot,
éthinylestradiol et
17β-estradiol valérate
et le progestatif est choisi dans le groupe des composés
diénogest,
gestodène,
lévonorgestrel,
désogestrel,
3-cétodésogestrel,
drospironénone,
cyprotérone acétate,
norgestimate et
noréthistérone, ainsi que
l'oestrogène du second composant hormonal est choisi dans le groupe des composés
17β-estradiol,
éthinylestradiol et
17β-estradiol valérate.

19. Nécessaire de contraception selon l'une quelconque des revendications précédentes 11 à 18, **caractérisé en ce que** l'oestrogène de la première phase est contenu dans chaque dose unitaire journalière à une dose de
1,0 à 6,0 mg de 17β-estradiol,
0,015 à 0,025 mg d'éthinylestradiol,
1,0 à 4,0 mg de 17β-estradiol valérate
et le progestatif est contenu dans chaque dose unitaire journalière à une dose de
1,0 à 3,0 mg de diénogest,
0,05 à 0,075 mg de gestodène,
0,05 à 0,125 mg de lévonorgestrel,
0,06 à 0,15 mg de désogestrel,
0,06 à 0,15 mg de 3-cétodésogestrel,
1,0 à 3,0 mg de drospironénone,
1,0 à 2,0 mg de cyprotérone acétate,
0,2 mg à 0,3 mg de norgestimate,
0,35 à 0,75 mg de noréthistérone.

20. Nécessaire de contraception selon l'une quelconque des revendications précédentes 11 à 19, **caractérisée en ce que** dans la seconde phase est contenue dans chaque dose unitaire journalière une quantité de
1,0 à 6,0 mg de 17β-estradiol,
0,002 à 0,04 mg d'éthinylestradiol,
1,0 à 4,0 mg de 17β-estradiol valérate.

21. Nécessaire de contraception contenant au moins 30 doses unitaires journalières contenant chacune au moins une substance active hormonale avec une première phase et une seconde,
qui contient dans sa première phase, en tant que substance active hormonale, en association, une préparation d'oestrogène et d'un progestatif à une dose au moins suffisante pour l'inhibition de l'ovulation, en configuration monophasique, et dans la seconde phase, en tant que substance active hormonale, seulement une préparation d'oestrogène, la première phase comprenant au moins 25 et au maximum 77 doses unitaires journalières, administrées en mode continu ou discontinu, et la seconde phase comprenant 5, 6 ou 7 doses unitaires journalières administrées en mode continu ou discontinu, et le nombre total des doses unitaires étant égal au nombre total des jours du cycle désiré, d'une longueur d'au moins 30 et d'au maximum 84 jours,
les doses unitaires de la première phase se trouvant en partie dans des sous-unités se répétant périodiquement, qui sont séparées les unes des autres dans l'espace et/ou par d'autres marques.

22. Nécessaire de contraception selon la revendication 21, **caractérisé en ce que** les doses unitaires se trouvent en sous-unités au plus tôt à partir de la 26^{ème} dose unitaire journalière.

23. Nécessaire de contraception selon la revendication 21 ou 22, **caractérisé en ce que** les sous-unités individuelles peuvent être séparées les unes des autres par des perforations ou d'autres dispositifs appropriés à la séparation.

24. Nécessaire de contraception selon l'une quelconque des revendications 21, 22 et 23, **caractérisé en ce que** les sous-unités séparées contiennent chacune 7 doses unitaires.

25. Nécessaire de contraception selon l'une quelconque des revendication précédentes 21 à 24, **caractérisé en ce que** la première phase comprend 28 plus 7 ou 28 plus un multiple de 7 doses unitaires journalières.

26. Nécessaire de contraception selon l'une quelconque des revendications précédentes 21 à 25, **caractérisé en ce que** la seconde phase comprend 7 doses unitaires journalières.

27. Nécessaire de contraception selon l'une quelconque des revendications précédentes 21 à 26, **caractérisé en ce que** l'oestrogène de la première phase est choisi dans le groupe des composés
17β-estradiol,
éthinylestradiol et
17β-estradiol valérate
et le progestatif est choisi dans le groupe des composés
diénogest,
gestodène,
lévonorgestrel,
désogestrel,
3-cétodésogestrel,
drospironénone,
cyprotérone acétate,
norgestimate et
noréthistérone, ainsi que
l'oestrogène du second composant hormonal est choisi dans le groupe des composés
17β-estradiol,
éthinylestradiol et
17β-estradiol valérate.

28. Nécessaire de contraception selon l'une quelconque des revendications précédentes 21 à 27, **caractérisé en ce que** l'oestrogène de la première phase est contenu dans chaque dose unitaire journalière à une dose de
1,0 à 6,0 mg de 17β-estradiol,
0,015 à 0,025 mg d'éthinylestradiol,
1,0 à 4,0 mg de 17β-estradiol valérate
et le progestatif est contenu dans chaque dose unitaire journalière à une dose de
1,0 à 3,0 mg de diénogest,
0,05 à 0,075 mg de gestodène,
0,05 à 0,125 mg de lévonorgestrel,
0,06 à 0,15 mg de désogestrel,
0,06 à 0,15 mg de 3-cétodésogestrel,
1,0 à 3,0 mg de drospironénone,
1,0 à 2,0 mg de cyprotérone acétate,
0,2 mg à 0,3 mg de norgestimate,
0,35 à 0,75 mg de noréthistérone.

29. Nécessaire de contraception selon l'une quelconque des revendications précédentes 21 à 28, **caractérisée en ce que** dans la seconde phase est contenue dans chaque dose unitaire journalière une quantité de
1,0 à 6,0 mg de 17β-estradiol,
0,002 à 0,04 mg d'éthinylestradiol,
1,0 à 4,0 mg de 17β-estradiol valérate.
